# EUROPÄISCHE PATENTANMELDUNG

(11) **EP 4 122 513 A1**
(43) Veröffentlichungstag der Anmeldung: **25.01.2023**
(21) Anmeldenummer: 21187086.0
(22) Anmeldetag: 22.07.2021
(51) Int. Cl.: A61M 5/315, A61M 5/24

(54) **VERBESSERTE BETÄTIGUNGSVORRICHTUNG FÜR EINEN PEN**

(71) Anmelder: Ypsomed AG, 3401 Burgdorf (CH)
(72) Erfinder: Brügger, Martin, 3065 Bolligen (CH); Hirschel, Jürg, 3007 Bern (CH); Hostettler, Patrick, 3415 Hasle (CH)
(74) Vertreter: Meier Obertüfer, Jürg

(57) **Zusammenfassung**

Die Erfindung betrifft eine Betätigungsvorrichtung (1) für eine Verabreichungsvorrichtung sowie eine Verabreichungsvorrichtung zur parenteralen Verabreichung eines Medikaments, aufweisend:
- ein Drehelement (9), welches relativ zu einem Gerätegehäuse (7) um einen zu einem Dosisvolumen proportionalen Winkel um die Achse drehbar ist
- ein relativ zum Gerätegehäuse (7) bewegbares Schubelement (11), welches auf einen Antrieb wirkt
- ein Magnetelement (14), welches so mit dem Drehelement (9) und/oder dem Schubelement (11) wirkverbunden ist, dass es dessen/deren Bewegungen relativ insbesondere zum Gerätegehäuse (7) mitmacht
- eine Sensoranordnung (20) aus zumindest drei Magnetfeldsensoren bestehend, wobei jeder dieser Magnetfeldsensoren eine Richtwirkung bezüglich der Richtung einer magnetischen Feldkomponente aufweist
- eine Auswertvorrichtung (30),
wobei zumindest ein erster und ein zweiter der Magnetfeldsensoren so angebracht sind, dass sie selektiv die Änderungen der Feldkomponente in Umfangrichtung erfassen und der zumindest dritte Magnetfeldsensor so angebracht ist, dass er selektiv die Änderungen der Feldkomponente in Axialrichtung erfasst.

## Beschreibung

### TECHNISCHES GEBIET

Die vorliegende Erfindung betrifft das Gebiet der Verabreichungsgeräte und Verabreichungssysteme, für welche das Erzeugen, Auswerten, Speichern und Kommunizieren von Daten relevant ist. In Ausführungsformen bzw. Weiterbildungen bezieht sich die Erfindung auf Vorrichtungen, Systeme und Verfahren für die zuverlässige und genaue Erfassung und Auswertung von Daten im Zusammenhang mit der benutzungsfreundlichen und sicheren Dosierung und Verabreichung von Medikamenten.

### HINTERGRUND DER ERFINDUNG

Die Offenbarung der vorliegenden Erfindung bezieht sich vor allem auf Verabreichungsvorrichtungen welche eine gewindegetriebene Kolbenstange aufweisen, die durch einen rotierenden Antrieb vorgetrieben werden kann. Solche Verabreichungsvorrichtungen werden beispielsweise für die Behandlung von Diabetes durch Verabreichung von Insulin benutzt.

WO2018/160425 A1 beschreibt ein Modul oder ein System zur Erfassung der verabreichten Dosen eines Medikaments mittels einem Pen. Die Offenbarung bezieht sich auf ein Dosiserkennungssystem zur Verwendung in Kombination mit einer Medikamentenabgabevorrichtung. In einem Aspekt umfasst das System ein Modul, das mit der Vorrichtung gekoppelt wird und einen Sensor enthält, der die relative Winkelposition eines an der Medikamentenabgabevorrichtung angebrachten Elements erfassen kann. Das System bestimmt die Art des Medikaments, das in der Vorrichtung enthalten ist, und/oder die Art der Vorrichtung, basierend auf der erfassten Winkelposition des abgetasteten Elements. In einem anderen Aspekt wird das Dosiserkennungssystem in Kombination mit einer Medikamentenabgabevorrichtung verwendet, die ein Dosiseinstellelement enthält, das sich während der Dosisabgabe relativ zu einem Stellglied dreht. Das Dosiserkennungssystem umfasst einen Sensor und ist in der Lage, die relativen Winkelpositionen eines an dem Dosiseinstellelement befestigten abgetasteten Elements während der Dosisabgabe zu erfassen und die abgegebene Dosismenge zu bestimmen. Verwandte Verfahren werden ebenfalls offengelegt. Eine quantitative Erfassung der verabreichten Dosis erfolgt mit mehreren Sensoren und einem Magnetring, welcher sich während der Verabreichung relativ zu den Sensoren bewegt. Während der Dosisvorwahl erfolgt bei diesem System keine Erfassung.

WO2019/001919 A1 beschreibt eine Zusatzvorrichtung, die dazu geeignet ist, lösbar an einer Medikamentenabgabevorrichtung angebracht zu werden, wobei die Zusatzvorrichtung dazu geeignet ist, wenn sie an einem Gehäuse der Medikamentenabgabevorrichtung angebracht ist, einen Betrag der Bewegung eines Anzeigeelements zu bestimmen, wobei die Zusatzvorrichtung eine Typenidentifizierungseinrichtung umfasst, die dazu geeignet ist, eine vordefinierte Typenidentifizierung an der Medikamentenabgabevorrichtung zu erfassen. Die Zusatzvorrichtung ist ferner so ausgelegt, dass sie von einem anfänglichen Nichtbetriebszustand in einen permanenten Betriebszustand aktiviert werden kann, der einem vordefinierten Zustand entspricht, wobei der vordefinierte Zustand einem identifizierten vordefinierten Typenbezeichner entspricht. Während der Dosisvorwahl erfolgt bei dieser Zusatzvorrichtung keine Erfassung.

Die WO2019/170429 zeigt ein Arzneimittelabgabesystem, umfassend: ein längliches Gehäuse, das eine Einstellstruktur zum Einstellen einer Dosis eines Arzneimittels über eine Winkelposition enthält; und eine Sensorstruktur zum Bestimmen der Dosis, die einen Magneten und einen magnetischen Sensor enthält, die so angeordnet sind, dass mindestens eine von einer Winkelposition und einer Verschiebung des magnetischen Sensors relativ zu dem Magneten als eine Funktion des elektrischen Widerstands des magnetischen Sensors bestimmt werden kann. Die Sensorstruktur ist in Bezug auf die Einstellstruktur so angeordnet, dass die Winkelposition der Einstellstruktur als eine Funktion der Winkelposition und/oder der Verschiebung des Magnetsensors bestimmt wird. Es wird auch eine Sensorstruktur beschrieben, die Folgendes umfasst: eine flexible Folie mit einem magnetischen Sensor in einer zylindrischen Formkonfiguration, die eine Achse umfasst; und einen Magneten, der an einer Linie parallel zu oder kollinear mit der Achse angeordnet ist. Die offenbarten Sensorstrukturen basieren auf dem magnetoresistiven Effekt und sind blosse Alternativen zu optischen oder potentiometrischen Messanordnungen wo Widerstandsänderungen analog ausgewertet oder interpoliert werden. Es bleibt offen, wie zwischen Verschiebung und Drehung differenziert werden kann oder wie fehlerfreie Ergebnisse sichergestellt werden können.

Der Begriff "Produkt", "Medikament" oder "medizinische Substanz" umfasst im vorliegenden Zusammenhang jede fliessfähige medizinische Formulierung, welche geeignet ist zur kontrollierten Verabreichung mittels einer Kanüle oder Hohlnadel in subkutanes oder intramuskuläres Gewebe, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP 1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Der Begriff "distal" bezeichnet eine zum vorderen, einstechseitigen Ende der Verabreichungsvorrichtung beziehungsweise zur Spitze der Injektionsnadel hin gerichtete Seite oder Richtung. Demgegenüber bezeichnet die Angabe "proximal" eine zum hinteren, dem einstechseitigen Ende gegenüberliegenden Ende der Verabreichungsvorrichtung hin gerichtete Seite oder Richtung.

Unter den Begriffen "Injektionssystem" oder "Injektor" wird in der vorliegenden Beschreibung eine Vorrichtung verstanden, bei der die Injektionsnadel nach erfolgter Abgabe einer kontrollierten Menge der medizinischen Substanz aus dem Gewebe entfernt wird. Somit verbleibt bei einem Injektionssystem oder bei einem Injektor im Unterschied zu einem Infusionssystem die Injektionsnadel nicht über einen längeren Zeitraum von mehreren Stunden im Gewebe.

### DARSTELLUNG DER ERFINDUNG

Es ist eine Aufgabe der vorliegenden Erfindung eine Betätigungsvorrichtung für eine Verabreichungsvorrichtung zur parenteralen Verabreichung eines Medikaments bereitzustellen, wobei die Betätigungsvorrichtung eine nur wenige zur berührungslosen Messung notwendige Einzelteile aufweisende Messeinrichtung beinhalten soll. Ferner soll eine hohe Genauigkeit, Fehlersicherheit, unkomplizierter Bedienung und ein geringer Energieverbrauch gewährleistet sein und eine Störanfälligkeit vermindert und die Herstellkosten reduziert werden.

Die Aufgabe wird gelöst durch eine Betätigungsvorrichtung für eine Verabreichungsvorrichtung zur parenteralen Verabreichung eines Medikaments nach Anspruch 1. Vorteilhafte Weiterbildungen gehen aus den abhängigen Ansprüchen hervor.

Eine Verabreichungsvorrichtung zur parenteralen Verabreichung eines Medikaments, insbesondere ein AutoPen oder ein Injektionsgerät, wie es der vorliegenden Erfindung zugrunde liegt, weist verschiedene mechanische Einrichtungen wie etwa eine Dosier- und eine Ausschütteinrichtung auf, welche aus mehreren Elementen aufgebaut sind. Beispielsweise ist zur Ausschüttung eines Produkts eine Kolbenstange z.B. in Form einer Gewindestange mit Flansch entlang der Längsachse des Injektionsgeräts relativ zu einem Produktbehälter, einem Gerätegehäuse oder weiteren Führungs- und/oder Antriebselementen antreibbar. Es kann eine Antriebsbewegung manuell beispielsweise über einen Gewindetrieb oder durch einen Motor, insbesondere einen Federmotor bewirkt werden.

Eine Verabreichungsvorrichtung zur parenteralen Verabreichung eines Medikaments kann eine erfindungsgemässe Betätigungsvorrichtung aufweisen. Zur Einstellung eines Dosisvolumens für ein zu verabreichendes Produkt dient z.B. ein hülsen- oder zylinderförmiges Drehelement mit einer Achse, welches Drehelement relativ zum Gerätegehäuse und koaxial um die Längsachse des Injektionsgeräts um einen zum vorgewählten und/oder ausgeschütteteten Dosisvolumen proportionalen Winkel um die Achse gedreht werden kann. Das Drehelement kann beispielsweise über einen Drehknopf von aussen drehbar sein und kann so mit dem Federmotor kuppelbar sein, dass dieser über die Drehung spannbar ist und/oder das Drehelement die Antriebsbewegung mitmacht. Weiter kann ein von aussen z.B. über einen Druckknopf betätigbares, entlang der Längsachse des Injektionsgeräts relativ zum Gerätegehäuse bewegbares Schubelement vorgesehen sein, welches auf den Motor oder eine Kupplung wirkt und die Antriebsbewegung auslöst oder freigibt bzw. sperrt. Das Schubelement kann das Drehelement drehbar und axial fest lagern womit letzteres die Schubbewegung mitmacht. Alternativ kann das Schubelement und das Drehelement einstückig ausgeführt sein und derart im Injektionsgerät gelagert sein, dass es sich sowohl relativ zum Gerätegehäuse drehen und entlang der Längsachse verschieben kann. Weiter kann das Schubelement unabhängig vom Drehelement kollinear oder koaxial zur Längsachse verschiebbar im Gerätegehäuse gelagert sein. Bevorzugt kann das Schubelement axial relativ zum Drehelement bewegbar und rotativ mit dem Drehelement gekoppelt sein. Das Schubelement kann derart bei Betätigung eine lineare, insbesondere axial gerichtete Lageänderung ausführen, wobei die Lageänderung durch axiale Endanschläge begrenzbar ist. Weiter kann das Schubelement gegen eine axial wirkende Federkraft einer Rückstellfeder betätigbar sein, welche jenes wieder in eine Ausgangsposition zurückbewegen kann. Das Drehelement führt bei einer drehenden Betätigung von aussen und/oder durch den Motor eine Winkeländerung aus, wobei die maximal mögliche Winkeländerung durch beispielsweise radiale Endanschläge begrenzbar ist. Weiter kann das Drehelement mit einer Rasteinrichtung wirkverbunden sein, welche das Drehelement in vorgegebenen, insbesondere regelmässig verteilten Rastpositionen bzw. Drehwinkeln kraftschlüssig halten kann. Beispielsweise kann diese Rasteinrichtung durch einen elastischen Schnapper in Form einer Ratsche oder durch eine federkraftbeaufschlagte Klickscheibe verwirklicht sein. Diese Rastpositionen bzw. Drehwinkel des Drehelements können vorgegebenen Dosisschritten des zu verabreichenden Medikaments entsprechen.

Weiter ist an der erfindungsgemässen Betätigungsvorrichtung eine Messvorrichtung vorgesehen, welche ein Magnetelement mit einer permanenten Magnetisierung aufweist, insbesondere ein hohlzylinder- oder torusförmiges Magnetelement, beispielsweise in Form einer Hohlwelle. Das Magnetelement kann sektorweise abwechselnd, insbesondere regelmässig verteilt magnetisiert sein, dass die Polwinkel bezüglich einer Drehachse alle gleich gross sind. Beispielsweise kann die permanente Magnetisierung jedes Sektors radial gerichtet sein, wobei sich auf den Mantelaussenflächen und den zugehörigen Mantelinnenflächen der Hohlzylindersektoren jeweils ein N- bzw. S-Pol ausbildet. Entsprechend sind auch an den in axiale Richtung weisenden Stirnflächen der Hohlzylindersektoren jeweils Gebiete des N- bzw. S-Pol ausgebildet. Alternativ kann die Magnetisierung auch als sogenannter Halbach-Array erfolgen mit dem Vorteil, dass sich der magnetische Fluss auf der Innenseite des Magnetelements fast aufhebt und auf der Aussenseite entsprechend verstärkt. Die Drehachse des Magnetelements kann vorteilhaft mit der Längsachse des Injektionsgeräts und/oder der Achse des Drehelements zusammenfallen. Beispielsweise kann das Magnetelement als Ringmagnet über den Umfang verteilt 10 Pole respektive Sektoren bzw. Polwinkel aufweisen, wobei bei regelmässiger Verteilung alle 36° ein N- auf einen S-Pol folgt. Auch mehr oder weniger Pole können vorgesehen sein, vorzugsweise 2 bis 40 oder mehr. Das Magnetelement kann so mit dem Drehelement und/oder dem Schubelement wirkverbunden sein, dass es dessen/deren Bewegungen relativ insbesondere zum Gerätegehäuse mitmacht. Beispielsweise ist das Magnetelement am Drehelement drehfest koaxial gelagert und gegenüber dem Schubelement axialfest aber drehbar verbunden oder das Magnetelement ist fest mit dem Schubelement verbunden, welches die Drehbewegung des Drehelements mitmacht. Das Magnetelement kann formschlüssig mit dem Drehelement oder dem Schubelement verbindbar sein, beispielsweise mittels Rippen und Rillen. Durch die Magnetisierung findet sich im Raum über der äusseren Oberfläche des Magnetelements ein unterschiedlich starkes und unterschiedlich gerichtetes magnetisches Feld. Einerseits bildet sich entlang des Umfangs gerichtet, in anderen Worten in Umfangrichtung, insbesondere quer bzw. tangential zur Drehachse gerichtet, eine abwechselnd polarisierte Flussdichte bzw. Feldkomponente mit einem Maximum jeweils mittig über den axial verlaufenden Polgrenzen aus. Gleichzeitig bildet sich zur Drehachse gerichtet, insbesondere parallel zur Drehachse gerichtet, in anderen Worten in Axialrichtung, aber um einen halben Polwinkel phasenverschoben zur axial verlaufenden Polgrenze eine abwechselnd polarisierte Flussdichte bzw. Feldkomponente mit einem Maximum jeweils über den proximalen und distalen Stirnflächen aus.

Vorteilhaft kann die Rasteinrichtung auch magnetisch arbeiten, wobei zumindest ein drehfest mit dem Gerätegehäuse verbundener, geeignet geformter und magnetisierter und/oder magnetisierbarer Stator das Magnetelement bzw. das mit diesem drehfest verbundene Drehelement durch magnetische Kräfte in die Rastpositionen bewegen und halten kann. Diese Rastpositionen bzw. die Drehwinkel zwischen den Rastpositionen des Drehelements können vorgegebenen Dosisschritten des zu verabreichenden Medikaments entsprechen. Beispielsweise kann eine magnetisch arbeitende Rasteinrichtung für eine Betätigungs- oder eine Verabreichungsvorrichtung zur parenteralen Verabreichung eines Medikaments zusätzlich zu einem relativ zum Gerätegehäuse drehbaren Magnetelement folgende Bauformen für ein Statorelement aufweisen: Einen gehäusefesten Magneten geformt als voller oder partieller Ring aufweisend eine sektorweise identische bzw. komplementäre permanente Magnetisierung wie das Magnetelement, vorzugsweise proximal oder distal stirnseitig oder radial bzw. umfangseitig über einen Luftspalt auf das Magnetelement wirkend. Eine solche Anordnung rastet per doppeltem Pol- resp. Sektorwinkel. Alternativ zumindest ein gehäusefestes stabförmig oder gebogen geformtes weichmagnetisches Statorelement angebracht im Magnetfeld von zumindest einem Pol des Magnetelements und von diesem um einen Luftspalt beabstandet. Bevorzugt kann ein solches Statorelement beispielsweise als U-förmiges Joch aus weichmagnetischem Material ausgebildet sein, welches zwei unterschiedliche Pole des Magnetelements verbindbar macht. Solche weichmagnetischen Anordnungen rasten per einfachem Pol- resp. Sektorwinkel.

Weiter weist die Messvorrichtung an der erfindungsgemässen Betätigungsvorrichtung eine Sensoranordnung auf, welche aus zumindest drei Magnetfeldsensoren besteht. Jeder dieser Magnetfeldsensoren weist eine ausgeprägte Empfindlichkeit bzw. Richtwirkung bezüglich der Richtung und/oder Polarisierung des magnetischen Feldes zu einer als Schaltachse bezeichneten Sensorachse auf. Ein digitaler Ausgang mit Speicher oder Latch eines jeden Magnetfeldsensors wird eingeschaltet bzw. gesetzt, wenn entlang der Schaltachse eine N nach S polarisierte Flussdichte einen Einschaltwert erreicht oder überschreitet und ausgeschaltet bzw. zurückgesetzt, wenn eine S nach N polarisierte Flussdichte einen Ausschaltwert erreicht oder überschreitet. Alternativ wird eingeschaltet bzw. gesetzt, wenn entlang der Schaltachse eine S nach N polarisierte Flussdichte einen Einschaltwert erreicht oder überschreitet und ausgeschaltet bzw. zurückgesetzt, wenn eine N nach S polarisierte Flussdichte einen Ausschaltwert erreicht oder überschreitet. Dieses Verhalten der Magnetfeldsensoren ist als bipolare Schaltcharakteristik bekannt. Auch können alternativ Sensoren eingesetzt sein, welche ein analoges Ausgangsignal liefern, welches entsprechend ausgewertet werden kann. Bevorzugt weisen die Magnetfeldsensoren eine integrierte Anordnung auf, welche nach dem magnetoresistiven Prinzip arbeitet, besonders bevorzugt nach dem tunnelmagnetoresistiven Prinzip. Solche Bauelemente sind auch als TMR-Sensoren bekannt. TMR-Sensoren bieten auf Grund ihrer hohen Empfindlichkeit bezüglich der magnetischen Feldstärke Vorteile bezüglich Energieverbrauch und bezüglich des Freiheitsgrads der konstruktiven Anordnung der Elemente der erfindungsgemässen Betätigungsvorrichtung. Auch kann dank der hohen Empfindlichkeit der TMR-Sensoren auf Seltenerdmagnete verzichtet werden und es können preisgünstige, korrosionsbeständige Materialien für das Magnetelement zum Einsatz kommen, wie z.B. Ferrite.

Die Sensoranordnung ist mit einer Auswertvorrichtung verbunden oder verbindbar, insbesondere einer computer- bzw. softwareimplementierten Auswertvorrichtung z.B. einer Schaltung aufweisend einen Microcontoller und/oder ein Field Programmable Gate Array FPGA. Dabei sind die Ausgänge der zumindest drei Magnetfeldsensoren mit entsprechenden Eingängen der Auswertvorrichtung verbindbar, wobei insbesondere jeder Magnetfeldsensor je zumindest einen Ausgang aufweist, welcher je mit einem entsprechenden Eingang der Auswertvorrichtung verbindbar oder verbunden ist. Es sind zumindest ein erster und ein zweiter der Magnetfeldsensoren so angebracht, dass sie selektiv die Änderungen der Feldkomponente in Umfangrichtung erfassen und der zumindest dritte Magnetfeldsensor ist so angebracht, dass er selektiv die Änderungen der Feldkomponente in Axialrichtung erfasst. Diese selektive Erfassung nach Feldkomponenten erlaubt eine sichere und einfache Unterscheidung zwischen bzw. Diskriminierung von Drehbewegungen und Schubbewegungen. Die Auswertvorrichtung implementiert vorteilhaft zumindest einen ersten Dekoder, welcher zumindest den ersten und zweiten der Eingänge als quadraturencodiertes Signal auswerten kann um die Drehung des Drehelements quantitativ zu erfassen. Eine Signaländerung an einem zumindest dritten Eingang kann als Lageänderung des Schubelements insbesondere auch als Drehung des Drehelements dekodiert werden. Die Signaländerungen an den Eingängen können dabei direkt in Echtzeit oder über einen FIFO-Puffer zwischengespeichert zur weiteren Verarbeitung gelangen. Derart sind mit einer geeigneten State Machine oder Speicherlogik die Bewegungen bzw. Zustände der Betätigungsvorrichtung abbildbar bzw. nachbildbar und stehen als Zustandsinformation zur weiteren Auswertung zur Verfügung. Mit diesen Zustandsinformationen können insbesondere die Dosierung, Injektionsmengen und Injektionszeiten, insbesondere eingestellte Dosis, korrigierte Dosis und/oder ausgeschüttete Dosis berechnet werden. Auch können aufgrund bestimmter Signalzustände oder Signalabfolgen an den Eingängen Fehler der Betätigungsvorrichtung oder Signalfehler der Messvorrichtung entdeckt und die Funktionssicherheit der Betätigungsvorrichtung oder verbundener Systeme kann verbessert werden. Vorzugsweise implementiert die Auswertvorrichtung einen zweiten Dekoder welcher den dritten Eingang und einen vierten Eingang als quadraturencodiertes Signal auswerten kann um die Drehung des Drehelements quantitativ zu erfassen und zusammen mit dem ersten Dekoder die Präzision der Erfassung und Diskriminierung der Lageänderung des Schubelements verbessern. Weiter kann die Auswertvorrichtung einen Eingang aufweisen, welcher bei einer Signaländerung die Auswertvorrichtung aus einem Energiesparzustand weckt und weitere Systemteile einschalten kann.

Bevorzugt können die Magnetfeldsensoren über die Auswertvorrichtung ein- und ausgeschaltet werden. Vorzugsweise kann zumindest ein Magnetfeldsensor von der Auswertvorrichtung bezüglich seiner Abtastrate variiert oder umgeschaltet werden. Beispielsweise kann dadurch in einem Ruhezustand der Betätigungsvorrichtung nur einer der zumindest 3 Magnetfeldsensoren eingeschaltet und eine niedrige Abtastrate gewählt sein wodurch im Ruhezustand der Betätigungsvorrichtung ein niedriger Energieverbrauch resultiert. Geeignet hohe Abtastraten für den Betriebszustand der Betätigungsvorrichtung ergeben sich aus den maximal zu erwartenden Winkelgeschwindigkeiten im Betriebszustand der Betätigungsvorrichtung sowie der Anzahl der über den Umfang des Magnetelements verteilten Pole. Bevorzugt ist pro Quadrant eines Poldurchgangs mindestens eine Abtastung vorzusehen. Geeignet hohe Abtastraten können beispielsweise zwischen 1 kHz bis 100 kHz oder höher, bevorzugt 5 kHz bis 20 kHz, beispielsweise ungefähr 10 kHz betragen.

Ein erster und ein zweiter Magnetfeldsensor sind in einem unbetätigten Zustand der Betätigungsvorrichtung entlang eines über dem Magnetelement in Umfangrichtung ausgerichteten ersten axialen Abschnitts und um einen ersten Sensorwinkel bezüglich der Drehachse zueinander sowie radial zum Magnetelement beabstandet auf einer Sensorebene über dem Magnetelement angebracht, wobei die Schaltachsen des ersten und zweiten Magnetfeldsensors in Umfangrichtung weisen bzw. tangential zum ersten Abschnitt bzw. der Sensorebene ausgerichtet und gleichgerichtet oder entgegengerichtet sind oder/und zumindest einer von erstem oder zweitem Magnetsensor eine invertierte Schaltcharakteristik oder ein invertiertes Ausgangsignal aufweist.

In einer vorteilhaften Weiterbildung beträgt der erste Sensorwinkel weniger als ein Polwinkel oder weniger als ein Polwinkel vermehrt um ein ganzzahliges Vielfaches eines Polwinkels. Bevorzugt ist das Magnetelement so bezüglich der Magnetfeldsensoren positioniert und sind die Schaltachsen des ersten und zweiten Magnetfeldsensors so gerichtet oder deren Ausgänge so geschaltet, dass in den Rastposition des Drehelements oder des Magnetelements bzw. wenn sich der erste und der zweite Magnetfeldsensor in einem gleich polarisierten Magnetfeld befinden jeweils einer der beiden Ausgänge gesetzt und der andere der beiden Ausgänge zurückgesetzt ist. Dies erlaubt eine platzsparende Bauweise der Sensoranordnung, insbesondere eine flächensparende Sensoranordnung auf der Sensorebene mit optimaler Ausnützung des Magnetfelds und damit einen verbesserten Störabstand gegenüber Fremdfeldern.

Ein dritter Magnetfeldsensor ist in einem unbetätigten Zustand der Betätigungsvorrichtung entlang eines in Umfangrichtung verlaufenden radial zum Magnetelement beabstandeten zweiten axialen Abschnitts auf der Sensorebene angebracht, wobei dieser zweite axiale Abschnitt axial zum ersten Abschnitt versetzt ist und/oder diesen überlagert, wobei die Schaltachse des dritten Magnetfeldsensor in axiale Richtung weist bzw. quer, insbesondere orthogonal zu den Schaltachsen des ersten und zweiten Magnetfeldsensors ausgerichtet ist. Dabei ist der dritte Magnetfeldsensor bzw. der zweite axiale Abschnitt relativ zum Magnetelement so positioniert, dass bei einer axialen Bewegung des Schubelements und/oder bei einer Drehung des Drehelements sein Ausgang gesetzt resp. zurückgesetzt werden kann. Das Ausgangssignal des dritten Magnetfeldsensors kann zur Erfassung einer Lageänderung des Schubelements und/der zur Erfassung einer Winkeländerung des Drehelements durch die Auswertvorrichtung herangezogen werden. Insbesondere kann dieser dritte Magnetfeldsensor in einem Ruhezustand der Betätigungsvorrichtung eingeschaltet und eine niedrige Abtastrate gewählt sein. Bei einer signalisierten Lageänderung des Schubelements und/oder bei einer signalisierten Winkeländerung des Drehelements kann so die Auswertvorrichtung eine Betätigung der Betätigungsvorrichtung erkennen und entsprechend die übrige Sensoranordnung einschalten und eine für einen Betriebszustand der Betätigungsvorrichtung geeignet hohe Abtastrate wählen womit die Betätigungsvorrichtung vom energiesparenden Ruhezustand in den Betriebszustand gelangt. Durch die geeignet hohe Abtastrate im Betriebszustand der Betätigungsvorrichtung kann der dritte Magnetsensor Lageänderungen und Winkeländerungen fehlerfrei und mit geringster Zeitverzögerung erfassen. Geeignet hohe Abtastraten ergeben sich aufgrund der maximal zu erwartenden Winkelgeschwindigkeiten des Drehelements im Betriebszustand der Betätigungsvorrichtung sowie der Anzahl der über den Umfang des Magnetelements verteilten Pole. Bevorzugt ist pro Quadrant eines Poldurchgangs mindestens eine Abtastung vorzusehen. Geeignet hohe Abtastraten können beispielsweise zwischen 1 kHz bis 100 kHz oder höher, bevorzugt 5 kHz bis 20 kHz, beispielsweise ungefähr 10 kHz betragen. Für den Ruhezustand der Betätigungsvorrichtung kann die Abtastrate beispielsweise um mindestens einen Faktor 100 tiefer angesetzt werden, wodurch ein entsprechend geringerer Energieverbrauch des dritten Magnetfeldsensors resultiert und dennoch eine Zustandsänderung sicher und schnell genug erfasst werden kann.

In einer vorteilhaften Weiterbildung ist ein vierter Magnetfeldsensor auf dem zweiten Abschnitt und um einen zweiten Sensorwinkel bezüglich der Drehachse zum dritten Magnetsensor sowie radial zum Magnetelement beabstandet auf der Sensorebene über dem Magnetelement angebracht, wobei die Schaltachse des vierten Magnetfeldsensors in axiale Richtung weist bzw. quer, insbesondere orthogonal zu den Schaltachsen des ersten und zweiten Magnetfeldsensors ausgerichtet ist, wobei die Schaltachsen des dritten und vierten Magnetfeldsensors gleichgerichtet oder entgegengerichtet sind oder/und einer von drittem oder viertem Magnetsensor einen invertierten Ausgang aufweist. Dabei ist der vierte Magnetfeldsensor relativ zum Magnetelement so positioniert, dass bei einer axialen Bewegung des Schubelements und/oder bei einer Drehung des Drehelements sein Ausgang gesetzt resp. zurückgesetzt werden kann. Das Ausgangssignal des dritten Magnetfeldsensors kann zur Erfassung einer Lageänderung des Schubelements und/oder zur Erfassung einer Winkeländerung des Drehelements durch die Auswertvorrichtung herangezogen werden.

In einer vorteilhaften Weiterbildung sind bevorzugt zumindest einer aus erstem und zweitem Magnetfeldsensor mit zumindest einem aus drittem und viertem Magnetsensor auf der Sensorebene axial in einer Linie bzw. auf derselben Winkelposition bezüglich der Drehachse angebracht.

In einer vorteilhaften Weiterbildung beträgt der zweite Sensorwinkel weniger als ein Polwinkel oder weniger als ein Polwinkel vermehrt um ein ganzzahliges Vielfaches eines Polwinkels. Bevorzugt ist das Magnetelement so bezüglich der Magnetfeldsensoren positioniert und sind die Schaltachsen des dritten und vierten Magnetfeldsensors so gerichtet oder deren Ausgänge so geschaltet, dass in den Rastposition des Drehelements oder des Magnetelements bzw. wenn sich der dritte und der vierte Magnetfeldsensor in einem ungleich polarisierten Magnetfeld befinden jeweils einer der beiden Ausgänge gesetzt und der andere der beiden Ausgänge zurückgesetzt ist. Dies erlaubt eine platzsparende Bauweise der Sensoranordnung, insbesondere eine flächensparende Sensoranordnung auf der Sensorebene mit optimaler Ausnützung des Magnetfelds und damit einen verbesserten Störabstand gegenüber Fremdfeldern.

In einer vorteilhaften Weiterbildung kann der zweite Sensorwinkel den gleichen Wert haben wie der erste Sensorwinkel. Dies erlaubt eine platzsparende Bauweise der Sensoranordnung, insbesondere eine flächensparende Sensoranordnung auf der Sensorebene mit optimaler Ausnützung des Magnetfelds und damit einen verbesserten Störabstand gegenüber Fremdfeldern.

In einer vorteilhaften Weiterbildung kann der erste und dritte Magnetfeldsensor integriert einem ersten Gehäuse und/oder der zweite und vierte Magnetfeldsensor integriert einem zweiten Gehäuse sein.

In einer vorteilhaften Weiterbildung kann die Sensorebene flach oder gekrümmt und/oder abschnittsweise geknickt, insbesondere zylindermantelförmig gebogen sein. Dies erlaubt eine Optimierung der radialen Position der Magnetfeldsensoren und damit eine optimierte Ausnützung des Magnetfelds und damit einen verbesserten Störabstand gegenüber Fremdfeldern.

In einer vorteilhaften Weiterbildung kann die Sensorebene durch eine Leiterplatte und/oder Leiterfolie gebildet sein, welche insbesondere fest mit dem Gerätegehäuse oder dem Schubelement verbindbar ist.

Weiter kann ein fünfter Magnetfeldsensor entlang des ersten und/oder zweiten axialen Abschnitts auf der Sensorebene angebracht sein, wobei die Schaltachse des fünften Magnetfeldsensors in axiale Richtung weist bzw. quer, insbesondere orthogonal zu den Schaltachsen des ersten und zweiten Magnetfeldsensors ausgerichtet ist. Dabei kann der fünfte Magnetfeldsensor relativ zum Magnetelement so positioniert sein, dass bei einer axialen Bewegung des Schubelements und/oder bei einer Drehung des Drehelements sein Ausgang gesetzt resp. zurückgesetzt werden kann. Das Ausgangssignal des fünften Magnetfeldsensors kann zur Erfassung einer Lageänderung des Schubelements und/oder zur Erfassung einer Winkeländerung des Drehelements durch die Auswertvorrichtung herangezogen werden. Insbesondere kann dieser fünfte Magnetfeldsensor in einem Ruhezustand der Betätigungsvorrichtung eingeschaltet und eine niedrige Abtastrate gewählt sein. Insbesondere kann dieser fünfte Magnetfeldsensor in einem Ruhezustand der Betätigungsvorrichtung eingeschaltet und eine niedrige Abtastrate gewählt sein. Bei einer signalisierten Lageänderung des Schubelements und/oder bei einer signalisierten Winkeländerung des Drehelements kann so die Auswertvorrichtung eine Betätigung der Betätigungsvorrichtung erkennen und entsprechend die übrige Sensoranordnung einschalten und eine für einen Betriebszustand der Betätigungsvorrichtung geeignet hohe Abtastrate wählen womit die Betätigungsvorrichtung vom energiesparenden Ruhezustand in den Betriebszustand gelangt. Der fünfte Magnetfeldsensor kann ausschliesslich dieser Weckfunktion oder auch einer erweiterten Plausibilitätsprüfung zusammen mit den übrigen Magnetfeldsensoren dienen.

Weiter kann die Betätigungsvorrichtung vom Betriebszustand in den Ruhezustand gelangen, wenn der fünfte Magnetfeldsensor und/oder weitere aus den ersten bis vierten Magnetfeldsensoren eine vorbestimmte Zeitdauer keine Signaländerungen an den jeweiligen Ausgängen aufweisen.

Die beschriebene Messanordnung und ihre erfindungsgemässen Weiterbildungen erfassen die Drehung bzw. den Drehwinkel des Drehelements bei der Dosiseinstellung und/oder bei der Produktausschüttung relativ. Aus der logischen Abfolge der sensierten Lage des Schubelements bzw. der Betätigung des Druckknopfs und der sensierten Bewegung des Drehelements kann eine absolute Position der Drehung bzw. des Drehwinkels des Drehelements mit Bezug auf das Gerätegehäuse abgeleitet und festgelegt bzw. als Referenzposition gespeichert werden. Beispielsweise kann ein betätigter Druckknopf bei Abwesenheit einer Drehung des Drehelements während einer vorbestimmten Zeit als Position Null des Drehelements gedeutet werden. Weiter kann als Vorbedingung dazu eine Referenzfahrt beispielsweise über einen vorbestimmten Drehwinkel in einer vorbestimmten Drehrichtung erforderlich sein oder es kann initial oder nach Fehlerzuständen eine vorgegebene Betätigungsabfolge durch den Benutzer erforderlich sein. Alternativ können weitere Sensoren bzw. Endschalter das Erreichen und/oder Überfahren einer oder mehrerer absoluten Referenzpositionen signalisieren, insbesondere Positionen des Drehelements mit Bezug auf das Gerätegehäuse.

Es umfasst eine Weiterbildung der Erfindung auch eine Verabreichungsvorrichtung zur parenteralen Verabreichung eines Medikaments, aufweisend:
- eine erfindungsgemässe Betätigungsvorrichtung
- eine Kolbenstange in Form einer Gewindestange mit Flansch
- ein im Gerätegehäuse gehaltener Produktbehälter
wobei eine Antriebsbewegung der Kolbenstange entlang der Längsachse des Gerätegehäuses durch die Betätigungsvorrichtung vorwählbar und/oder steuerbar und manuell beispielsweise über einen Gewindetrieb oder automatisch durch einen Motor, insbesondere einen Elektromotor oder einen Federmotor bewirkbar ist, wodurch das Medikament aus dem Produktbehälter durch beispielsweise eine Nadel ausgetrieben wird.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden.
- Fig. 1: zeigt die Betätigungsvorrichtung in einem AutoPen im unbetätigten Zustand
- Fig. 2: zeigt die Betätigungsvorrichtung in einem AutoPen im betätigten Zustand
- Fig. 3: zeigt Bestandteile der Betätigungsvorrichtung in Explosionsdarstellung
- Fig. 4: zeigt das Magnetelement
- Fig. 5: zeigt die in magnetische Feldstärke By (mT) in Umfangrichtung als Abwicklung
- Fig. 6: zeigt die in magnetische Feldstärke Bx (mT) in Axialrichtung als Abwicklung
- Fig. 7: zeigt eine Aufsicht der Messanordnung im unbetätigten Zustand
- Fig. 8: zeigt einen Längsschnitt der Messanordnung im betätigten Zustand
- Fig. 9: zeigt die Leiterplatte mit Magnetfeldsensoren (von der Drehachse her gesehen)
- Fig. 10: zeigt die Lage des Magnetelements, der Sensoren und des Gerätegehäuses zueinander im unbetätigten Zustand
- Fig. 11: zeigt die Lage des Magnetelements, der Sensoren und des Gerätegehäuses zueinander im betätigten Zustand
- Fig. 12: zeigt ein State Event Diagramm für die zweifach quadraturencodierten Signale
- Fig. 13: zeigt ein Ablaufdiagramm zur Auswertung der zweifach quadraturencodierten Signale in einen aktuellen Zustand eines AutoPen
- Fig. 14: zeigt ein State Event Diagramm für das Einschalten

### FIGURENBESCHREIBUNG

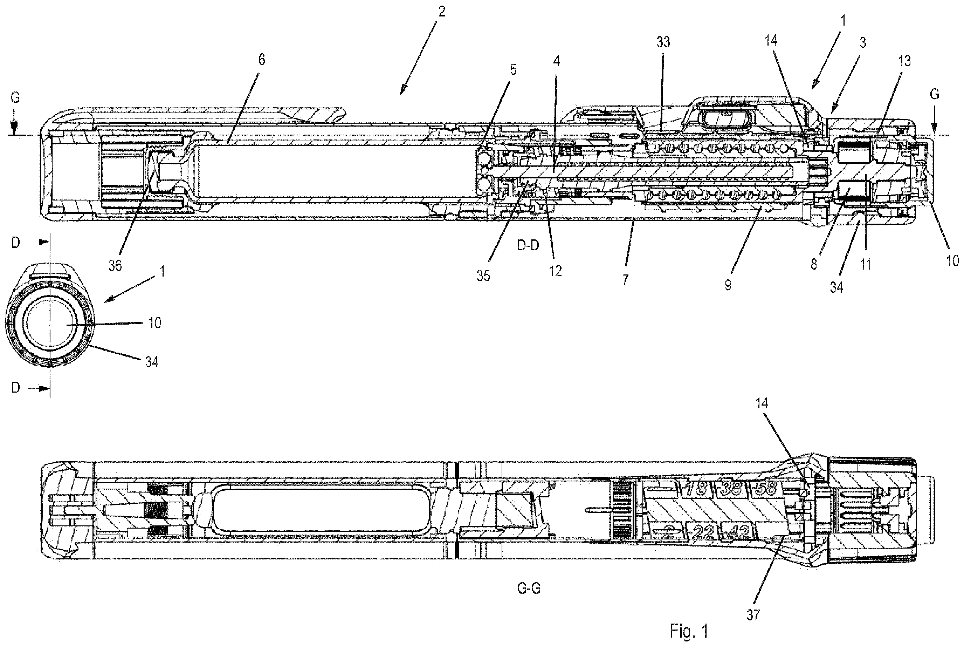

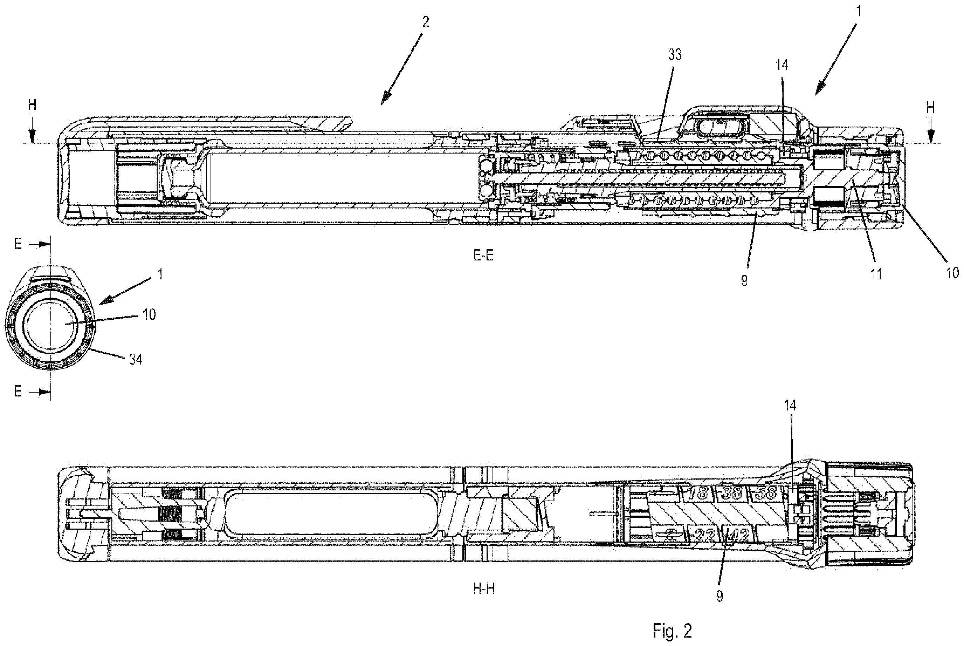

Figuren 1 bis 3 zeigen eine Ausführungsform der erfindungsgemässen Betätigungsvorrichtung 1 in einer Verabreichungsvorrichtung am Beispiel eines AutoPen 2. Der AutoPen 2 weist einen mit einem Medikament gefüllten Produktbehälter 6 auf welcher im Gerätegehäuse 7 gehalten ist. Mit einer vom Federmotor 8 antreibbaren Antriebshülse 35 kann die Kolbenstange mit dem Flansch 5 durch ein Gewinde in distale Richtung vorgetrieben werden, wobei ein Stopfen im Produktbehälter 6 das Medikament durch eine Kanüle (nicht gezeigt) im Septum 36 drängt bis ein sich mit der Antriebshülse 35 mitdrehendes Drehelement 9 am Gerätegehäuse 7 durch einen radialen Anschlag 37 zum Stillstand kommt. Das Drehelement 9 ist mit einer durch das Skalenfenster 33 ablesbaren Skala versehen. Zum Einstellen einer weiteren zu verabreichenden Medikamentenmenge oder Dosis kann das Drehelement 9 durch Drehen am Drehknopf 34 über eine Gewindeverbindung mit dem Gerätegehäuse 7 von diesem Anschlag 37 eine dosisproportionale Distanz bzw. einen Drehwinkel wegbewegt werden. Die Drehbewegung wird dabei vom Drehknopf 34 über das Schubelement 11 auf das Drehelement 9 übertragen, wobei die Drehbewegung den zwischen Schubelement 11 und Gerätegehäuse wirkenden Federmotor 8 spannt. Eine Rasteinrichtung 13 vermag das Drehmoment des Federmotors 8 in einem unbetätigten Zustand der Betätigungsvorrichtung in vorgegebenen Drehposition bzw. Dosisschritten zu halten. Durch Betätigen des Druckknopfs 10 wird das Schubelement 11 gegen die Kraft der Rückstellfeder 12 in distale Richtung bewegt, wodurch das Drehmoment des Federmotors 8 auf die Antriebshülse 35 umgekuppelt wird und diese zusammen mit Drehelement in Rotation versetzen kann. Wird der Druckknopf 10 losgelassen, wird das Schubelement 11 durch die Rückstellfeder 12 in seine proximale Ausgangslage zurückbewegt und das Drehmoment des Federmotors 8 wird wieder auf die Rasteinrichtung umgekuppelt. Beispielsweise ist eine solche Verabreichungseinrichtung in Form eines AutoPen offenbart in der Publikation WO2009/105910 A1 deren Lehre in diese Beschreibung durch Verweis vollumfänglich aufgenommen wird.

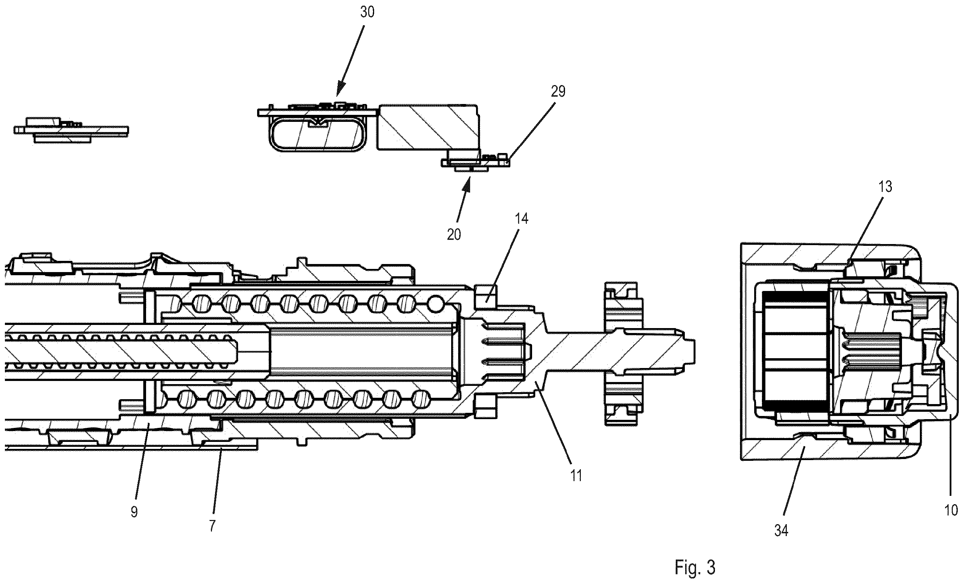

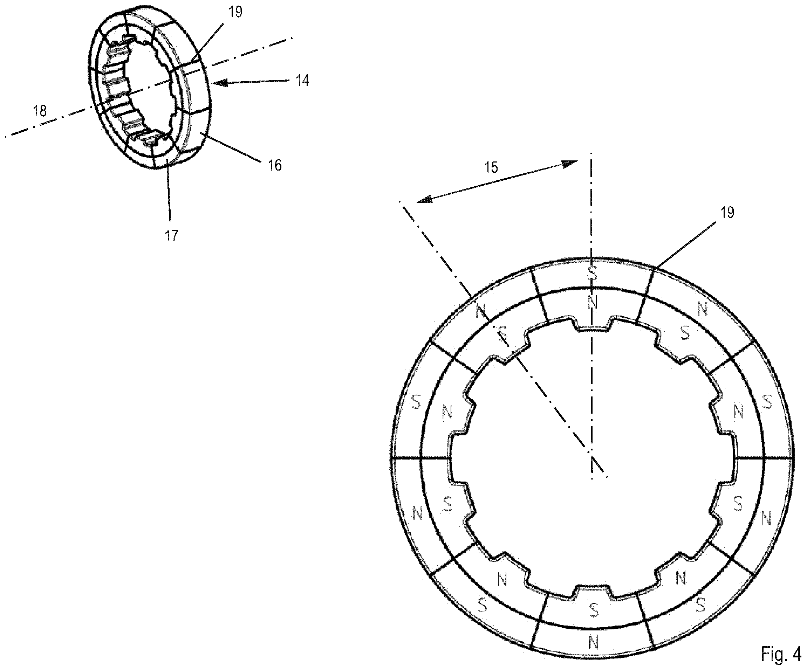

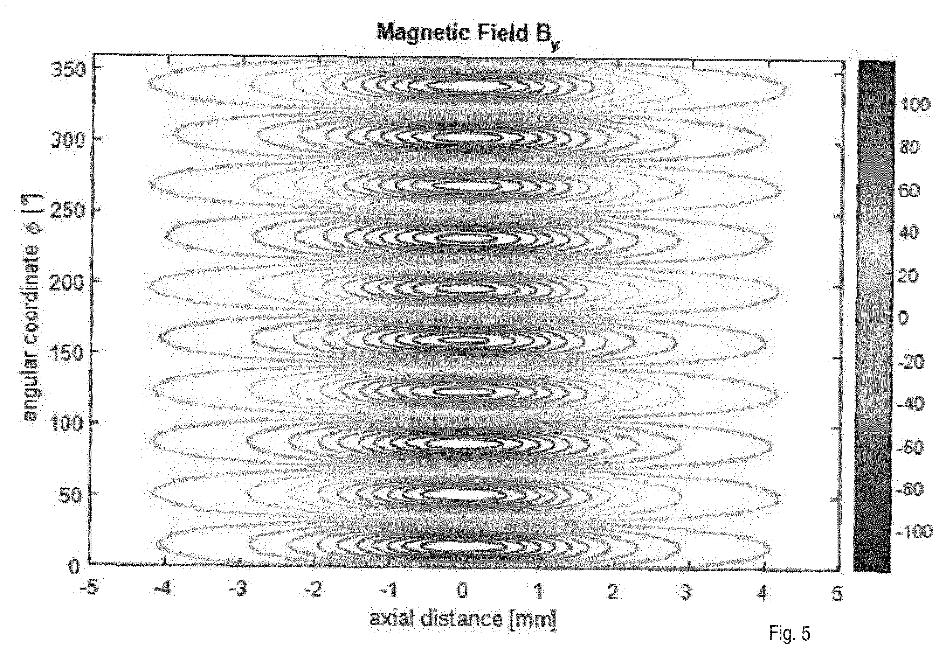

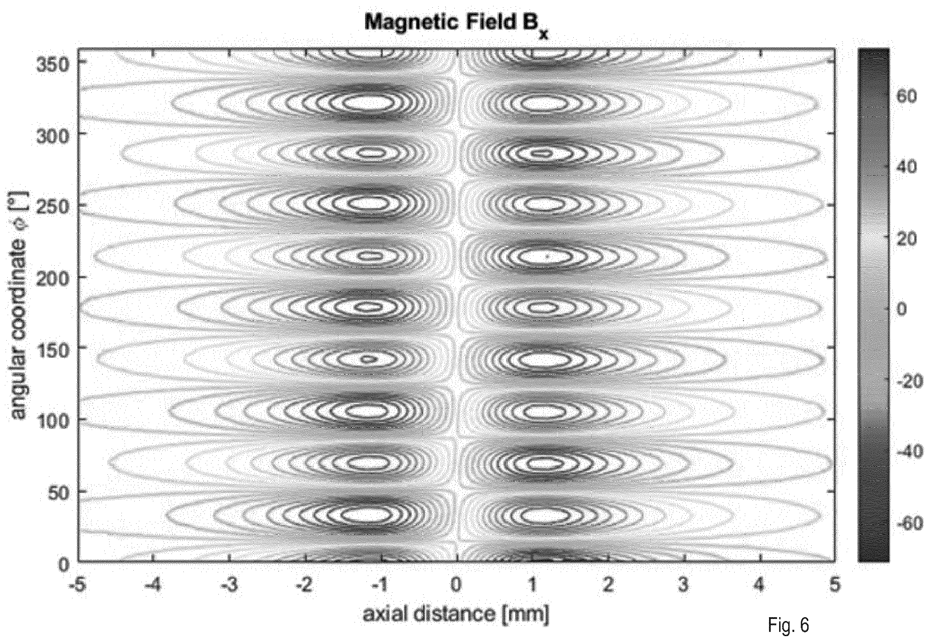

Die Ausführungsform der erfindungsgemäss verbesserten Betätigungsvorrichtung weist nun wie in Figur 3 gezeigt neben den oben beschriebenen Teilen Druckknopf 10, Drehknopf 34, Rasteinrichtung 13, Schubelement 11, Drehelement 9 und Gerätegehäuse 7 folgende Teile auf: ein auf dem Schubelement angebrachtes Magnetelement 14 in Form eines sektorweise magnetisierten Hohlzylinders aus einem Material mit permanentmagnetischen Eigenschaften, beispielsweise aus Ferrit. Eine Sensoranordnung 20 sowie eine Auswertvorrichtung 30. Figur 4 zeigt das Magnetelement 14 mit seiner Drehachse 18 welche mit der Längsachse der Drehelements 9 zusammenfällt, da das Magnetelement 14 mittels formschlüssigen Verbindungen koaxial auf jenem befestigbar ist. Dadurch macht das Magnetelement alle Bewegungen des Schubelements 11 mit. Eine Magnetisierung erfolgt sektoriell in radialer Richtung alternierend polarisiert, wodurch sich auf der Mantelaussenfläche 16 des Magnetelements 14, zehn um einen Polwinkel 15 beabstandete Pole ausbilden, welche durch Polgrenzen 19 getrennt sind. Das Magnetfeld zwischen benachbarten Polen ist über den Polgrenzen 16 ausgeprägt in Umfangrichtung gerichtet. Figur 5 zeigt die auf einer radial von der Mantelaussenfläche 16 beabstandeten koaxialen Ebene gemessene magnetische Feldstärke Bx in mT in Umfangrichtung als Abwicklung. Durch die Magnetisierung bilden sich auch auf der proximalen und der distalen Stirnfläche 17 entsprechende Pole aus. Dies Pole haben auf der genannten koaxialen Ebene über dem Magnetelement 14 jedoch ein ausgeprägt in axiale Richtung ausgerichtetes Magnetfeld zu Folge. Figur 6 zeigt die auf einer radial von der Mantelaussenfläche 16 beabstandeten koaxialen Ebene gemessene magnetische Feldstärke By in mT in axialer Richtung als Abwicklung. Die Maxima dieser Feldkomponente By sind gegenüber den Maxima der Feldkomponente Bx um einen halben Polwinkel phasenverschoben und befinden sich jeweils über der proximalen Stirnflächen 17.

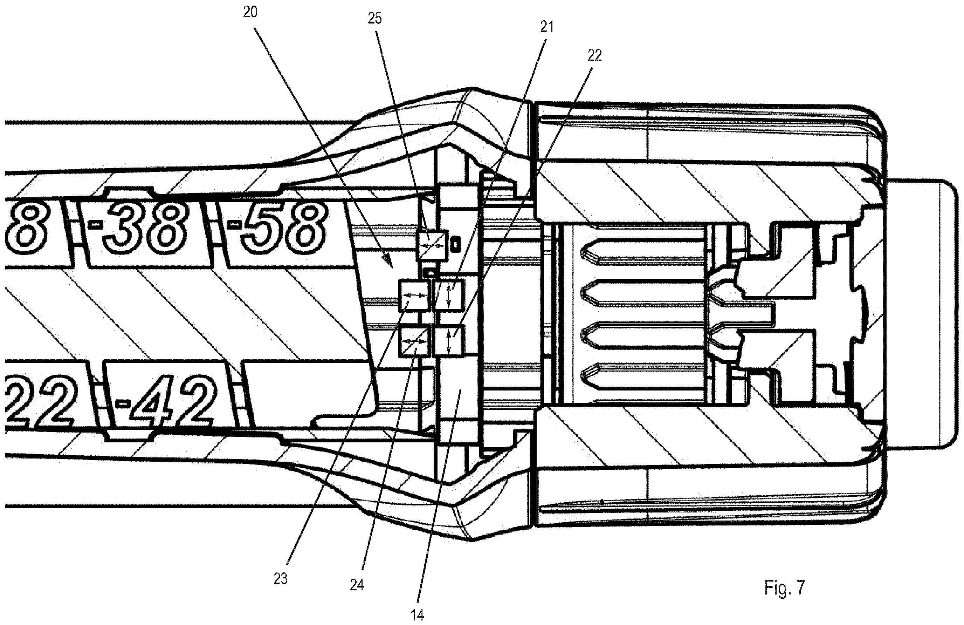

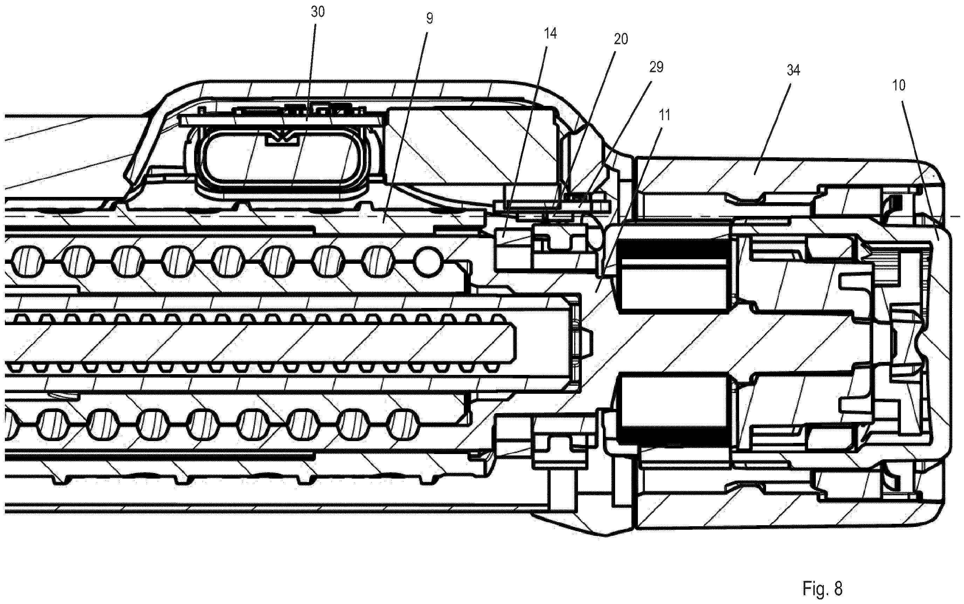

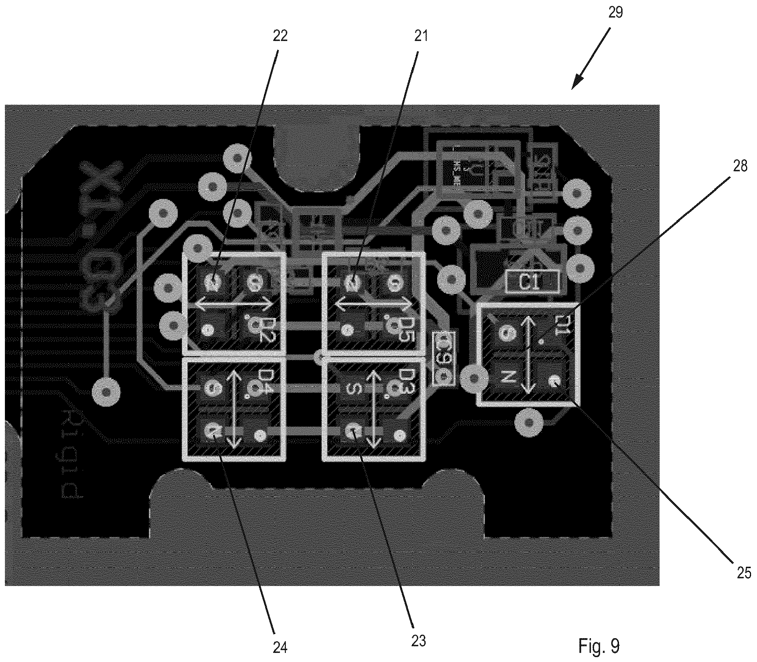

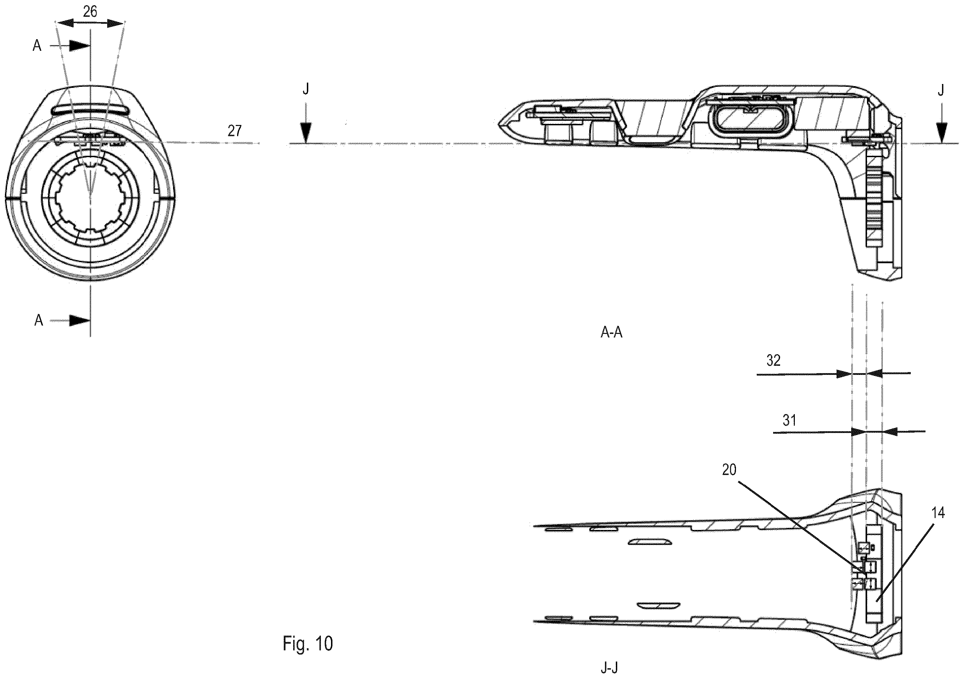

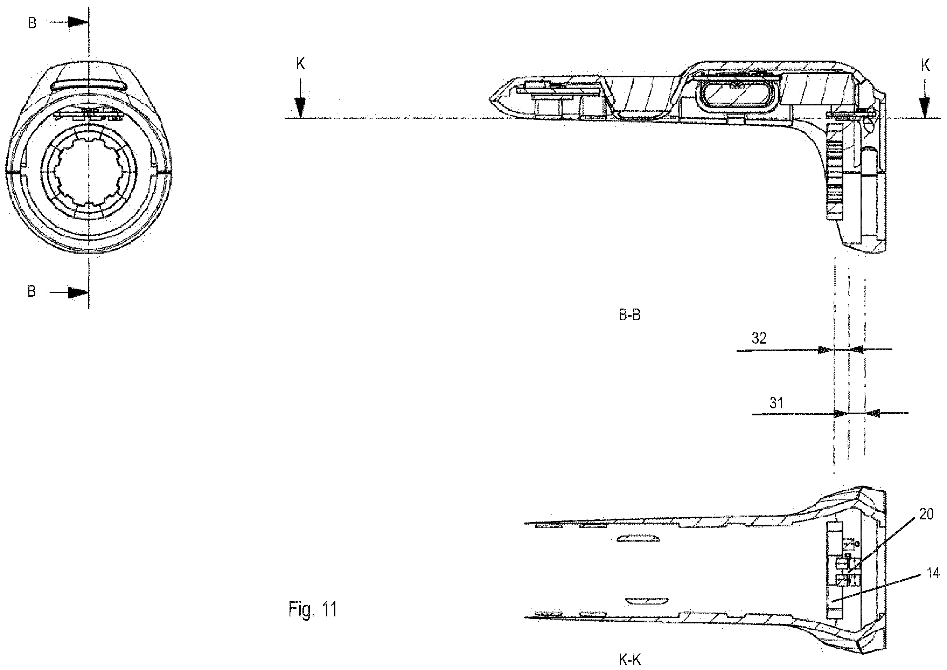

Figur 7 zeigt die Sensoranordnung 20 im unbetätigten Zustand der Ausführungsform und Figur 8 zeigt dieselbe Anordnung im betätigten Zustand der Ausführungsform in einem 90° gedrehten Längsschnitt. Dabei sind fünf Magnetfeldsensoren 21..25 auf einer Trägerplatte oder Leiterplatte 29 angeordnet. Ausführungsformen mit drei oder vier Sensoren ergeben sich aus der hier beschriebenen Ausführungsform mit fünf Sensoren und der allg. Erfindungsbeschreibung ohne weiteres. Es handelt sich bei den Magnetfeldsensoren 21...25 um TMR Sensoren mit bipolarer Schaltcharakteristik der Serie RR122 des Herstellers RedRock im LGA Gehäuse. Das Magnetelement 14 mit seiner Mantelaussenfläche 16 kann berührungslos gegenüber der gehäusefesten Sensoranordnung 20 um seine Drehachse gedreht und axial beweget werden. Dabei sind die Schaltachsen der Magnetfeldsensoren 21 und 22, welche folgend auch Sensor A und B genannt werden, so ausgerichtet, dass sie die Feldkomponente By sensieren, insbesondere ihren Ausgang schalten, wenn die Feldkomponente By durch eine Relativbewegung des Magnetelements 14 zum Sensor ihre Polarität wechselt. Die Schaltachsen der Magnetfeldsensoren 23 und 24, welche folgend auch Sensoren B und C genannt werden, sind so ausgerichtet, dass sie die Feldkomponente Bx sensieren, insbesondere ihren Ausgang schalten, wenn die Feldkomponente Bx durch eine Relativbewegung des Magnetelements 14 zum Sensor ihre Polarität wechselt. Dies gelingt, wenn die Sensoren beispielsweise wie in Figur 9 gezeigt auf der Leiterplatte 29 angeordnet sind. Die Sensoren A und B sensieren so nur eine Drehbewegung des Drehelements 9 unabhängig von dessen axialer Lage. Dagegen kommen die Sensoren B und C bei der Betätigung des Druckknopfs 10 und einer axialen Lageänderung des Drehelements 9 vom der Feldkomponente Bx über der distalen Stirnfläche 17 des Magnetelements 14 in die umgekehrt polarisierte Feldkomponente Bx über der proximalen Stirnfläche 17 des Magnetelements 14. Dadurch sensieren die Sensoren B und C sowohl eine Drehung wie auch eine axiale Lageänderung des Drehelements 9. Figuren 10 zeigt, wie in der hier beschriebenen Ausführungsform der erfindungsgemässen Betätigungsvorrichtung in die Sensoren A und B entlang eines axialen Abschnitts 31 und die Sensoren B und C entlang eines axialen Abschnitts 32 angebracht sind. Dabei sind die Sensorpaare A und B bzw. C und D unter einem Sensorwinkel 26 bezüglich der Drehachse 18 radial beabstandet über der Mantelfläche 16 angebracht, wobei der Sensorwinkel 26 kleiner ist als der Polwinkel 15. Der Ausgang des Sensors A ist invertiert oder dessen Schaltachse ist 180° gegenüber der Schaltachse von Sensor B gedreht. Dies kompensiert die Phasenverschiebung um einen halben Polwinkel der Feldkomponenten Bx und By und die Sensoren A,C sowie B,C können kompakt je an der gleichen Winkelposition platzsparend auf der Leiterplatte angebracht werden. Ein fünfter Magnetfeldsensor 25 ist tangential etwas weiter von Magnetfeldelement 14 entfernt auf der Leiterplatte 29 angebracht. Seine Schaltachse 28 ist so ausgerichtet, dass er durch Änderungen der Feldkomponente Bx seinen Ausgang schaltet, wenn die Feldkomponente Bx durch eine Relativbewegung des Magnetelements 14 zum Sensor ihre Polarität wechselt. Figur 11 zeigt die Anordnung aus Figur 10 in einem betätigten Zustand der Betätigungsvorrichtung.

Weiter zeigt Figur 8 die Auswertvorrichtung 30 auf deren Eingänge die entsprechenden Ausgänge der Magnetfeldsensoren führen. Die Auswertvorrichtung 30 kann die Magnetfeldsensoren zudem ein- oder ausschalten und/oder ihre Abtastrate ändern. Weiter kann die Auswertvorrichtung Anzeigeelemente wie LED oder Display ansteuern und/oder drahtlos mit weiteren Systemen eines Verabreichungssystems kommunizieren, beispielsweise mit einer Fernbedienung oder einem Mobiltelefon oder einem System, welches den Blutzucker misst und eine angepasste Dosierung bestimmt.

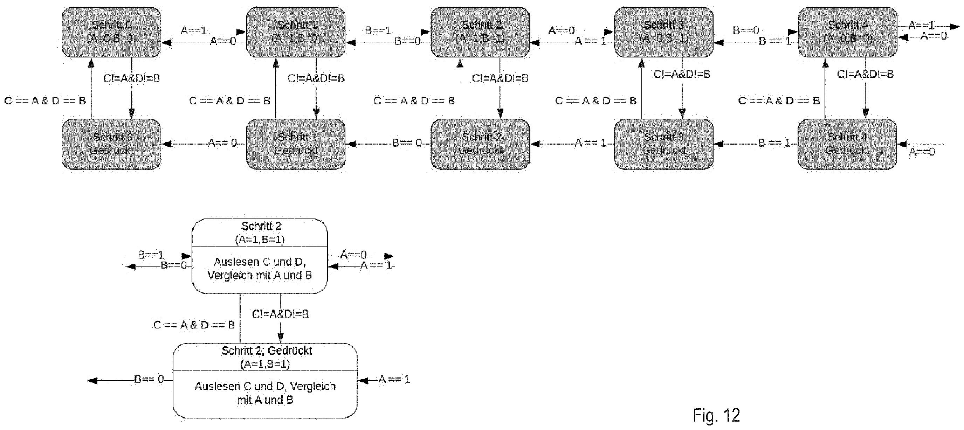

Figur 12 zeigt die State Machine für die zweifach quadraturencodierten Signale der Sensoren A/B und B/C. Es können durch diese Logik wie gezeigt durch Vergleich der Zustände bzw. Änderung der vier Signale über ein Intervall von vier Schritten jeweils die Drehrichtung, die relative Drehposition, der Betätigungszustand "gedrückt" sowie Fehlerzustände (nicht gezeigt) abgebildet werden.

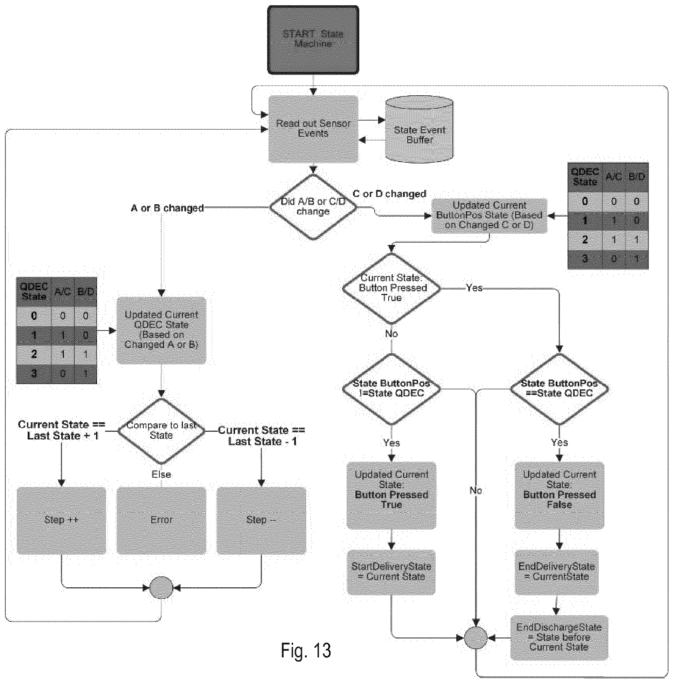

Fig. 13 zeigt ein Ablaufdiagramm zur Auswertung der zweifach quadraturencodierten Signale in einen aktuellen Zustand des AutoPen. Dazu wird eine State Machine implementiert um den aktuellen Pen Zustand des AutoPen wiederzugeben. Diese State Machine läuft nicht in Echtzeit. Es ist ein Event Buffer vorhanden, welcher die Sensorsignale speichert. Dabei werden die Sensorsignale per Interrupt in den Buffer geschrieben. Die Verarbeitung des Buffer läuft zyklisch beispielsweise alle 10ms. Das heisst die State Machine verarbeitet sequentiell alle Sensorsignale, welche in den letzten 10ms in den Buffer geschrieben wurden. Zusätzlich wird im Verarbeitungszyklus die State Machine zu verschiedenen Zuständen abgefragt und die relevanten Informationen abgeleitet. Dabei wird je nachdem ob ein QDEC Sensorevent (Sensoren A oder B) oder Druckknopfevent (Sensoren C und D) vorhanden ist entweder der QDEC State aktualisiert oder der Druckknopf Status überprüft. Der aktuelle Pen Zustand (Current State) wird durch die Schrittposition beschrieben und ob der Druckknopf gedrückt oder gelöst ist. Zusätzlich muss immer der Status des Quadraturschrittes (QDEC State: 0-3) für das QDEC- und ButtonPos-Signal bekannt sein. Der Zeitpunkt des aktuellen Schrittes (letzte Änderung Schrittposition oder Druckknopfposition) muss auch gespeichert sein. Der aktuelle Pen Zustand wird immer aktualisiert sobald ein Sensorevent ausgewertet wird. Wenn der Druckknopf gedrückt wird an einer Position grösser 0 (möglicher Start Ausschüttung), wird der StartDeliveryState aktiviert. Dabei wird der aktuelle Schritt und Zeitpunkt gespeichert. Wenn der Druckknopf wieder gelöst wird der EndDischargeState und EndDeliveryState aktiviert. Dabei ist der Zeitpunkt des EndDischargeStates das Erreichens des Drehschrittes (letzter Zeitpunkt vor Lösend des Druckknopfes) und der EndDeliveryState Zeitpunkt das lösen des Druckknopfes.

Mit den Informationen der verschiedenen delivery states können die Injektionsmenge und Injektionszeiten berechnet werden. Zusätzlich zur Erkennung des aktuellen Pen Zustand wird im Algorithmus überprüft ob ein Sensorik Fehler vorliegt. Anhand der Quadraturschritte können Fehler in den QDEC- und Druckknopfsignalen erkannt werden. Wenn im Algorithmus ein Fehler erkannt wird oder aus einem anderen Grund der letzte Pen Zustand nicht korrekt vorhanden ist (z.B. Akku entladen), geht die absolute Position des AutoPens verloren. Um die korrekte Position des Algorithmus wiederzufinden muss ein Reset der State Machine getätigt werden. Dabei muss der Benutzer miteingebunden werden um die absolute Nullposition wiederzufinden. Der Benutzer muss eine Betätigung ausführen, welche physikalisch eindeutig ist und nicht versehentlich anders ausgeführt werden kann. Beispielsweise muss der Benutzer den Drehknopf 34 von einer festgelegten Anzahl Schritte stetig abnehmend bis auf 0 drehen und dann den Druckknopf 10 drücken. Wenn so diese festgelegte Anzahl Schritte von der Auswerteinrichtung gezählt werden kann die korrekte Position bestimmt und der aktuelle Zustand des AutoPen als absolute Position festgelegt bzw. im Algorithmus abgebildet werden.

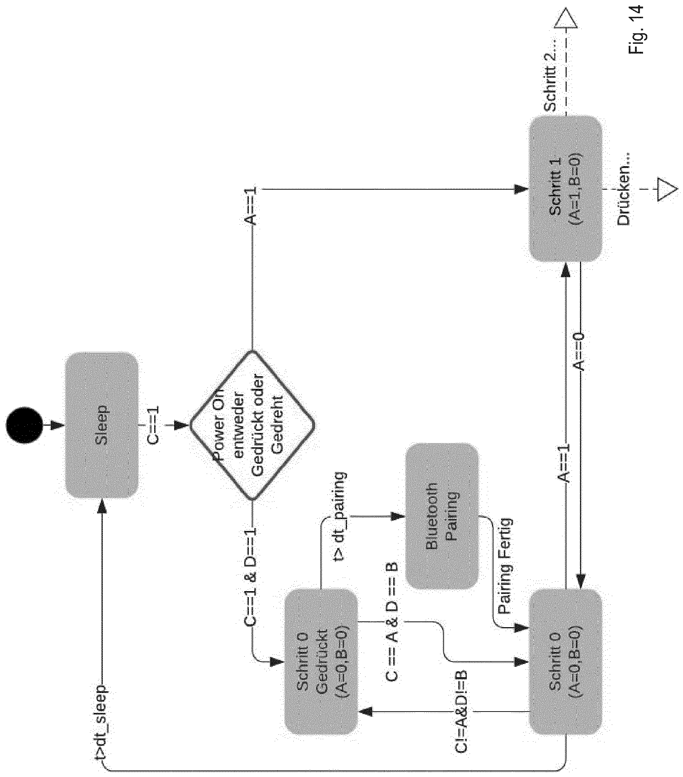

Figur 14 zeigt ein State Event Diagramm für das Einschalten des Pens. Das Aufwachen aus dem Sleep Mode wird durch den fünften Magnetfeldsensor (Startsensor) ausgelöst, welcher das gleiche Feld wie der TMR-Sensor C misst. Wenn die Sensorik aus dem Sleep-Mode gestartet wird darf kein Schritt verloren gehen. Das heisst wenn am Drehknopf 34 gedreht wird, muss der erste Schritt erkannt werden und wenn der Druckknopf 10 gedrückt wird muss auch dies rechtzeitig erkannt werden. Deshalb ist beispielsweise eine Abtastfrequenz von 500 Hz für den Startsensor nötig.

### BEZUGSZEICHENLISTE

- 1: Betätigungsvorrichtung
- 2: Verabreichungsvorrichtung
- 3: Messeinrichtung
- 4: Kolbenstange
- 5: Flansch
- 6: Produktbehälter
- 7: Gerätegehäuse
- 8: Federmotor
- 9: Drehelement
- 10: Druckknopf
- 11: Schubelement
- 12: Rückstellfeder
- 13: Rasteinrichtung
- 14: Magnetelement, Ringmagnet
- 15: Polwinkel
- 16: Mantelaussenfläche, Pol, Sektor
- 17: Stirnfläche, Pol, Sektor
- 18: Drehachse
- 19: Polgrenze
- 20: Sensoranordnung
- 21: erster Magnetfeldsensor, Sensor A
- 22: zweiter Magnetfeldsensor, Sensor B
- 23: dritter Magnetfeldsensor, Sensor C
- 24: vierter Magnetfeldsensor, Sensor D
- 25: fünfter Magnetfeldsensor
- 26: Sensorwinkel (erster oder zweiter)
- 27: Sensorebene
- 28: Schaltachsen der Magnetfeldsensoren
- 29: Leiterplatte
- 30: Auswertvorrichtung
- 31: erster axialer Abschnitt der Sensorebene
- 32: zweiter axialer Abschnitt der Sensorebene
- 33: Skalenfenster
- 34: Drehknopf
- 35: Antriebshülse
- 36: Septum
- 37: Anschlag
- A-A bis K-K: Längsschnitte

## Patentansprüche

1. Betätigungsvorrichtung (1) für eine Verabreichungsvorrichtung zur parenteralen Verabreichung eines Medikaments, aufweisend:
- ein hülsen- oder zylinderförmiges Drehelement (9) mit einer Achse, welches Drehelement (9) relativ zu einem Gerätegehäuse (7) und koaxial oder kollinear zu einer Längsachse der Betätigungsvorrichtung (1) um einen zu einem vorgewählten oder ausgeschütteten Dosisvolumen proportionalen Winkel um die Achse drehbar ist
- ein betätigbares, entlang der Längsachse der Betätigungsvorrichtung (1) relativ zum Gerätegehäuse (7) bewegbares Schubelement (11), welches auf einen Antrieb oder eine Kupplung wirkt
- ein Magnetelement (14) mit einer permanenten Magnetisierung, welches Magnetelement (14) so mit dem Drehelement (9) und/oder dem Schubelement (11) wirkverbunden ist, dass es dessen/deren Bewegungen relativ insbesondere zum Gerätegehäuse (7) mitmacht
- eine Sensoranordnung (20) aus zumindest drei Magnetfeldsensoren bestehend, wobei jeder dieser Magnetfeldsensoren eine ausgeprägte Empfindlichkeit bzw. Richtwirkung bezüglich der Richtung einer magnetischen Feldkomponente zu einer als Schaltachse (28) bezeichneten Sensorachse aufweist
- eine Auswertvorrichtung (30), wobei jeder Magnetfeldsensor je zumindest einen Ausgang aufweist, welcher je mit einem entsprechenden Eingang der Auswertvorrichtung verbindbar oder verbunden ist
**dadurch gekennzeichnet, dass**
zumindest ein erster und ein zweiter der Magnetfeldsensoren so angebracht sind, dass sie selektiv die Änderungen der Feldkomponente in Umfangrichtung erfassen und der zumindest dritte Magnetfeldsensor so angebracht ist, dass er selektiv die Änderungen der Feldkomponente in Axialrichtung erfasst.

2. Betätigungsvorrichtung (1) nach Anspruch 1, wobei das Schubelement (11) über einen Druckknopf (10) betätigbar ist und/oder das Drehelement (9) über einen Drehknopf (34) und/oder über einen Motor (8) drehbar ist.

3. Betätigungsvorrichtung (1) nach einem der vorhergehenden Ansprüche, wobei das Magnetelement (14) sektorweise abwechselnd, insbesondere regelmässig verteilt magnetisiert ist, so dass die Polwinkel (15) bezüglich einer Drehachse (18) alle gleich gross sind

4. Betätigungsvorrichtung (1) nach einem der vorhergehenden Ansprüche, wobei der erste und der zweite Magnetfeldsensor (21,22) in einem unbetätigten Zustand der Betätigungsvorrichtung (1) entlang eines über dem Magnetelement (14) in Umfangrichtung ausgerichteten ersten axialen Abschnitts (31) und um einen ersten Sensorwinkel (26) zueinander sowie radial zum Magnetelement (14) beabstandet auf einer Sensorebene (27) angebracht sind und wobei die Schaltachsen (28) des ersten und zweiten Magnetfeldsensors (21,22) in Umfangrichtung weisen bzw. tangential zum ersten axialen Abschnitt (31) bzw. der Sensorebene (27) ausgerichtet sind und wobei die Schaltachsen (28) des ersten und zweiten Magnetfeldsensors (21,22) gleichgerichtet oder entgegengerichtet sind

5. Betätigungsvorrichtung (1) nach dem vorhergehenden Anspruch, wobei der erste Sensorwinkel (26) weniger als ein Polwinkel (15) oder weniger als ein Polwinkel (15) vermehrt um ein ganzzahliges Vielfaches eines Polwinkels (15) beträgt

6. Betätigungsvorrichtung (1) nach einem der vorhergehenden Ansprüche, wobei der dritte Magnetfeldsensor (23) in einem unbetätigten Zustand der Betätigungsvorrichtung (1) entlang eines in Umfangrichtung verlaufenden radial zum Magnetelement (14) beabstandeten zweiten axialen Abschnitts (32) auf der Sensorebene angebracht ist, wobei dieser zweite axiale Abschnitt (32) axial zum ersten Abschnitt (31) versetzt ist und/oder diesen überlagert, wobei die Schaltachse (28) des dritten Magnetfeldsensor (23) in axiale Richtung weist oder quer zu den Schaltachsen (28) des ersten und zweiten Magnetfeldsensors (21,22) ausgerichtet ist

7. Betätigungsvorrichtung (1) nach dem vorhergehenden Anspruch, wobei ein vierter Magnetfeldsensor (24) auf dem zweiten Abschnitt (32) und um einen zweiten Sensorwinkel zum dritten Magnetsensor (23) sowie radial zum Magnetelement (14) beabstandet auf der Sensorebene (27) über dem Magnetelement (14) angebracht ist, wobei die Schaltachse (28) des vierten Magnetfeldsensors (24) in axiale Richtung weist oder quer zu den Schaltachsen (28) des ersten und zweiten Magnetfeldsensors (21,22) ausgerichtet ist, wobei die Schaltachsen (28) des dritten und vierten Magnetfeldsensors (23,24) gleichgerichtet oder entgegengerichtet sind

8. Betätigungsvorrichtung (1) nach dem vorhergehenden Anspruch, wobei der zweite Sensorwinkel weniger als ein Polwinkel (15) oder weniger als ein Polwinkel (15) vermehrt um ein ganzzahliges Vielfaches eines Polwinkels (15) beträgt

9. Betätigungsvorrichtung (1) nach einem der vorhergehenden Ansprüche, wobei der zweite Sensorwinkel den gleichen Wert hat wie der erste Sensorwinkel (26)

10. Betätigungsvorrichtung (1) nach einem der vorhergehenden Ansprüche 4 bis 9, wobei die Sensorebene (27) flach oder gekrümmt und/oder abschnittsweise geknickt, insbesondere zylindermantelförmig gebogen ist

11. Betätigungsvorrichtung (1) nach einem der vorhergehenden Ansprüche, wobei die Auswertvorrichtung (30) zumindest einen ersten Dekoder implementiert, welcher zumindest den ersten und zweiten der Eingänge als quadraturencodiertes Signal auswerten kann um die Drehung des Drehelements quantitativ zu erfassen

12. Betätigungsvorrichtung (1) nach dem vorhergehenden Anspruch, wobei eine Signaländerung an dem zumindest dritten Eingang der Auswertvorrichtung (30) als Lageänderung des Schubelements (11) und/oder als Drehung des Drehelements (9) dekodiert werden kann

13. Betätigungsvorrichtung (1) nach einem der vorhergehenden Ansprüche 11 bis 12, wobei die Auswertvorrichtung (30) einen zweiten Dekoder implementiert, welcher den dritten und vierten der Eingänge als quadraturencodiertes Signal auswerten kann um die Drehung des Drehelements (9) quantitativ zu erfassen

14. Betätigungsvorrichtung (1) nach einem der vorhergehenden Ansprüche 7 bis 13, wobei ein fünfter Magnetfeldsensor (25) entlang des ersten und/oder zweiten axialen Abschnitts (31,32) angebracht ist und die Auswertvorrichtung (30) einen weiteren Eingang aufweist, welcher bei einer Signaländerung die Auswertvorrichtung (30) aus einem Energiesparzustand weckt

15. Verabreichungsvorrichtung (2) zur parenteralen Verabreichung eines Medikaments, aufweisend:
- eine Betätigungsvorrichtung (1) nach einem der vorhergehenden Ansprüche
- eine Kolbenstange (4) in Form einer Gewindestange mit Flansch (5)
- ein im Gerätegehäuse (7) gehaltener Produktbehälter (6)
wobei eine Antriebsbewegung der Kolbenstange (4) entlang der Längsachse des Gerätegehäuses (7) durch die Betätigungsvorrichtung (1) vorwählbar und/oder steuerbar und manuell beispielsweise über einen Gewindetrieb oder automatisch durch einen Motor, insbesondere einen Federmotor (8) bewirkbar ist, wodurch das Medikament aus dem Produktbehälter durch beispielsweise eine Nadel ausgetrieben wird.
